# EUROPEAN PATENT APPLICATION

(11) **EP 4 803 899 A1**
(43) Date of publication of application: **09.09.2026**
(21) Application number: 25386015.9
(22) Date of filing: 05.03.2025
(51) Int. Cl.: G01N 33/00, G01N 33/02

(54) **SWEET TASTE ASSAY**

(71) Applicant: Special Account for Research Funds of University of Crete (SARF UoC), 74150 Rethimnon (GR); Justus Liebig University Giessen, 35390 Giessen (DE)
(72) Inventor: Ioannis, Pavlidis, 70013 Crete (GR); Theodoridi, Antonia, 70013 Crete (GR); Thomai, Lazou, 70013 Crete (GR); Gand, Martin, 35396 Gießen (DE)

(57) **Abstract**

A method for identification and/or measuring of sweetness of compounds comprising the steps a) providing larvae of a species of the family of Drosophilidae and/or of the family of Ephydroidea; b) providing an observation chamber having two separate regions, whereat the first region is to receive a reference compound and the second region is to receive the sample which is to be tested for sweetness or be measured for its contend of a sweet compound; c) populating the observation chamber with the larvae provided according to step a) and placing the reference compound and the sample each one into the region ascertained to it within the observation chamber, whereat the succession of populating with larvae, placing reference compound and placing sample is arbitrary; d) observing the behavior of the larvae as to within which of the two separate regions they prefer to stay; e) calculating a quantitative value from the observations of step d), thereby providing either a distinct decision if the sample exerts sweetness or providing a numerical value of concentration of a sweet compound within the sample.

## Description

The invention is about the establishment and validation of a sweet taste assay to identify potential new sweet compounds, especially sweet proteins.

The global imperative to reduce sugar consumption due to its association with health issues has fueled the search for alternative sweeteners. While artificial sweeteners have been prevalent, concerns about their health effects have shifted attention to natural sweet proteins. Sweet proteins, known since the isolation of miraculin in 1968, have been underutilized despite their immense potential. Identified proteins such as thaumatin, monellin, mabinlin, lysozyme, pentadin, brazzein, curculin, and miraculin exhibit sweetness values hundreds or thousands of times higher than sucrose. These proteins are sourced from tropical plants or, in the case of lysozyme, from egg white. While structural characteristics of these proteins alone don't fully explain the sweetness of these proteins, studies reveal that the interaction with the human sweet taste receptor, a heterodimeric G-protein-coupled receptor, is the molecular basis for their sweetness. Due to the absence of easily accessible human sweet taste receptor alternatives, such as electronic tongues and noses, other options must be considered for identifying potential new sweet proteins. The invention revealed herein comprises a *Drosophila melanogaster* larval assay as an innovative method for assessing the sweetness of new compounds, especially new proteins. It can also be used for quantitatively measuring the concentration of already known sweet compounds, resp. sweet proteins. The assay's effectiveness was demonstrated by evaluating heterologously expressed known sweet proteins. Notably, an enhanced thermal-stable monellin variant, MNEI, and Mabinlin, expressed in *Escherichia coli,* were successfully purified and subjected to assessment, using the developed assay. This approach provides a valuable platform for evaluating the sweetness of proteins and/or other compounds, especially in the absence of readily available human receptor substitutes. The utilization of *D. melanogaster* larvae, exhibiting a taste perception akin to humans, enhances the applicability and relevance of this assay for sweet protein evaluation.

### Introduction

In recent years, the correlation of high sugar consumption and diseases, such as type II diabetes, obesity, and poor oral health, has prompted a global trend to reduce sugar intake. Consequently, food manufacturers are constantly exploring alternative sweeteners (food additives that can mimic the sweetness of sugar) to create more cost-effective food and beverage products with low to zero calories that meet safety standards.

To date, this trend has mainly relied on the use of artificial or natural non-nutritive sweeteners. However, artificial sweeteners, like aspartame, have been associated with adverse side effects on health, including dizziness, headaches, gastrointestinal issues, mood changes, psychological problems, and bladder cancer, leading to reduced public acceptance in recent years. As a result, both research and industry focus has shifted towards natural sweeteners, such as sweet proteins, which are low in calories and not linked to detrimental health effects.

Sweet proteins are among the least explored natural sweeteners, even though the first sweetener protein, miraculin, was isolated in 1968. Their sweetening effect of all known sweet proteins is hundreds or thousands of times higher compared to sucrose on a molar basis, which is the universal reference substance with a sweetness value of 1. To date, eight sweet and taste-modifying proteins have been identified, namely, thaumatin, monellin, mabinlin, lysozyme, pentadin, brazzein, curculin (neoculin), and miraculin. All these proteins have different 3-dimensional (3D) structures and origins.

These proteins are isolated mostly from tropical plants, except for lysozyme, which occurs naturally in egg white. Plant-based proteins are categorized into two categories, taste-modifying proteins, and sweet-tasting proteins. The latter ones are extremely sweet, while both are low-caloric. The most extensively studied proteins of this family are monellin, thaumatin, and brazzein. Thaumatin is an intensely sweet-tasting protein isolated from the katemfe fruit, also known as miracle fruit, of *Thaumatococcus daniellii* Benth (Marantaceae) plant originating from West Africa. Thaumatin elicits a sweet taste sensation at approximately 50 nM, which can be retained for about half an hour. Also, it shows a licorice aftertaste based on the interactions with bitter taste receptors (taste receptors type 2, T2Rs). Thaumatin sweetening effect is nearly 3.000-fold sweeter than sucrose. It consists of a single chain of 207 amino acid residues (AAs) with eight intramolecular disulfide bonds. The 3D structure of thaumatin has been solved by X-ray studies and reveals that the structure contains three domains: I) an 11-stranded *β-*sandwich folded into two Greek key motifs in which all beta-strands are antiparallel besides the *N*- and C-terminal domain. This sweet protein exists at least in five different variants: two major (thaumatin I and II), and three minors (thaumatin a, b, and c), with molecular weight around 22 kDa and isoelectric point (pl) about 12. Furthermore, it is the only sweet protein available on a commercial scale with the name Talin. Talin is an approved sweetener in more than 20 countries by different risk assessment authorities. The United States Food and Drug Administration (FDA) for example approved thaumatin as "Generally Recognized As Safe" (GRAS) for about 30 applications. In the European Union (EU) thaumatin is approved by the European Food Safety Authority (EFSA) as both flavor and enhancer sweetener with the number E957.

Brazzein and Pentadin were isolated from the African plant Oubli, *Pentadiplandra brazzeana* Bailon. They are 500- to 2,000-times sweeter than sucrose, with a threshold value of around 500 nM. Brazzein has a pure sweet sensation, without a sour, salty, or bitter, but a lingering aftertaste thus, it is culturally considered to be safe to consume although it has not been formally recognized with GRAS status by the FDA. Also, brazzein is the smallest sweet-tasting protein (6.5 kDa) with a high pH and thermal stability. This sweet protein consists of 54 AAs and has four intramolecular disulfide bonds. The structural characterization reveals two forms of brazzein; the major, which contains a pyroglutamate residue (pGlu-brazzein) at its N-terminal, and the minor form (des-pGlu1-brazzein) without this residue. NMR spectroscopy reveals that the full-length brazzein consists of one short *α*-helix (residues 21-29) and three *β*-strands [residues (5-7), (34-39), (44-50)], which form a triple-stranded antiparallel *β*-sheet. Another unique tertiary characteristic of brazzein is the "cysteine-stabilized alpha-beta" motif (CSαβ), which is involved in the stabilization of an *α*-helix with the closest *β*-strand by forming two disulfide bonds.

Native monellin is isolated from the fruit, known as the serendipity berry, of the tropical plant *Dioscoreophyllum cumminsii* Diels in West Africa. It is a small, alkaline protein (approximately 11 kDa, pl -9.3) with a cystatin-like fold and it consists of two non-covalent polypeptide chains. Chain A contains of 44 AAs and chain B has 50 AAs linked together by non-covalent interactions. While the chains individually are not sweet, in their native form, they exhibit an estimated sweetness value of around 100,000-fold, and leaving an aftertaste for over an hour. However, the chains dissociate when the protein is heated above 50°C. To enhance the thermal stability of monellin, MNEI was constructed by linking both subunits through a dipeptide (Gly-Phe), preserving the sweetness at a similar value.

Mabinlin-II is another small, alkaline heterodimer protein (approximately 18 kDa, pl around 11.3), isolated from the mature seeds of mabinlang (*Capparis masaaki* Levl.) and grow in the subtropical region Yunnan of China. These seeds elicit a sweet perception, 400 times sweeter than sucrose, with a long-lasting aftertaste. Structurally, chain A consists of 33 AAs residues, while chain B has 72 AAs residues and both are connected by two intermolecular disulfide bridges. The identification of these bridges was elucidated by solving the 3D structure, which also clarified that the encoded precursor of mabinlin-II is a single chain consisting of 155 AAs. This single chain comprises a signal peptide of 20 residues, an *N*-terminal extension peptide of 15 residues, a linker peptide of 14 residues, and a single-residue *C*-terminal extension.

However, solely solving the structures of sweet proteins doesn't provide an answer to why these proteins are recognized as sweeteners. Therefore, different studies have illustrated that the molecular reason for the sweetness of those proteins is based on the interaction with the sweet taste receptor, which is a heterodimeric class C G-protein-coupled receptors (GPCRs) consisting of the subunits T1R2 (taste receptor type 1, member 2) and T1R3 (taste receptor type 1, member 3). Those taste receptor cells (TRCs) are located in taste buds on the tongue.

All members of class C GPCRs share distinct structural characteristics comprising a large extracellular *N*--terminal domain (NTD) linked to the seven transmembrane-spanning core domain (7TMD) by a well-conserved cysteine-rich domain (CRD). The use of site-directed mutagenesis studies and molecular modeling have shown that the sweet-taste receptor can identify different classes of sweet molecules ranging from sugar such as sucrose or glucose, to amino acids and proteins. However, due to their large molecular size compared to the small sugar molecules, the sweetener proteins do not fit into the same interacting area, suggesting that the sweet-taste receptor has multiple binding sites. One of the few theories that seem to explain the interaction between the larger sweet proteins with the T1R2-T1R3 heterodimer is the so-called "wedge model". According to this model, sweet proteins bind to an external cavity on the exterior of the heterodimer, and the shape and charges of the surfaces between the sweet protein and the receptor modulate their interaction.

However, merely solving the structures of sweet proteins doesn't provide a complete understanding of why these proteins are recognized as sweeteners. Therefore, herein, a newly established sweet taste assay employing the fruit fly *Drosophila melanogaster* as model organism, is used to determine sweetness, and this is validated using sweet protein models expressed in *Escherichia coli.* This invention allows the identification of novel putative sweet proteins and reduces the need for large-scale taste experiments, involving trained panelists, in the quest for new sweet variants.

### Materials and methods

### Bacterial strains and growth conditions

The synthetic gene encoding for the target proteins was purchased from GenScript (Piscataway, NJ, USA) and cloned in the pET21a(+) vector, using Ndel and Xhol as restriction enzymes. For the recombinant expression, 5 mL of an overnight pre-culture (37°C and 160 rpm) of a single colony of *E*. *coli* with the construct of interest was used to inoculate 500 mL of Lysogeny Broth (LB; 0.5% Yeast Extract (Condalab, Madrid, Spain), 1.0% Tryptone (AcumediaLab, Bury, UK) and 1.0% NaCl (Riedel-de Haën, Hannover, Germany) medium containing 100 µg/mL ampicillin (Sigma-Aldrich, St. Louis, MO, USA). Initially, different *E. coli* strains were screened (BL21(DE3), C41, C43, SHuffle T7), while *E. coli* BL21(DE3) gave the best results for MNEI and SHuffle T7 for Mabinlin-II, these strains were chosen for the further experiments. The use of chaperons from the TAKARA chaperon plasmid set (TAKARA Bio, Tokyo, Japan) did not improve the yield. The culture was incubated at 37°C with orbital shaking at 120 rpm until the OD at 600 nm (OD₆₀₀) reached a value around 0.6-0.8. After that, the induction was achieved with 0.2 mM isopropyl-β-D-1-thiogalactopyranoside (IPTG, SERVA, Heidelberg, Germany) and left for 16-18 h at 20°C and 120 rpm for expression. The culture was harvested by centrifugation at 6,000 x *g* for 20 min at 4°C, the supernatants were discarded and the pellet was collected and stored at -20°C.

### Cell lysis and protein purification

Cells were resuspended in different lysis buffers for better solubility of the final protein. MNEI was resuspended in phosphate buffer (K₂HPO₄/KH₂PO₄, 50 mM, pH 8) (Carl Roth Karlsruhe, Germany) with 300 mM NaCl and 15 mM imidazole. The mabinlin-II was resuspended in phosphate buffer (K₂HPO₄ /KH₂PO₄, 50 mM, pH 8) with 100 mM NaCl, 20% glycerol, and 0.1% Triton-X-100 (Sigma-Aldrich). Cells were lysed by sonication using an ultrasonic sonicator Sonifier 250 (Branson sonic power company, Danbury, USA), at 30% amplitude, 50% pulse, for five cycles of 30 s sonication, following 30 s of resting in ice.

The suspension was then centrifuged at 6,000 x *g* for 20 min at 4°C and the supernatant containing the soluble His-tagged proteins, filtered with 0.22 µm PVDF filters (GE Healthcare UK Limited, Buckinghamshire, UK), and used for purification by affinity chromatography, using ÄKTA Start Protein Purification System controlled from the UNICORN 7.0 software (Cytiva Europe GmbH, Freiburg, Germany) with a Ni-NTA HisTrap HP 5 mL column (Cytiva Europe GmbH, Freiburg, Germany). The system was equilibrated using 95% of buffer A (phosphate buffer, 50 mM, pH 8 with 300 mM NaCl) and 5% of buffer B (Buffer A + 300 mM imidazole) with 3 mL/min flow. Afterward, the column was loaded with the sample with 1.5 mL/min flow and washed four times using 90% buffer A and 10% buffer B with 3 mL/min flow. The proteins were eluted with 100% buffer B. Desalting was performed in Amicon^{®} Ultra Centrifugal Filters (Merck Millipore Ltd., Cork, Ireland) with molecular mass cut-off (MWCO) of 10 kDa using sodium phosphate (50 mM, pH 8). Finally, the desalted protein was aliquoted and flash-frozen with liquid nitrogen, while sodium dodecyl sulfate-polyacrylamide gel electrophoresis (SDS-PAGE) and protein quantification (via Bradford assay) were performed to determine the purity and concentration of the produced protein. Lysozyme (SERVA, Heidelberg, Germany) was used as a reference for molecular mass. Samples were stored at -20°C until use.

### Rearing conditions of flies and diet

For the sweet taste assay, the *Drosophila melanogaster* strain Oregon R was used. Flies were housed and bred in the quarantine facility. The population was reared under a 12 h light (L): 12 h dark (D) photoperiod at 21 ± 2°C and 65 ± 5% relative humidity. Insects were kept in sterile vials, which contained a cornmeal diet that consisted of agar (5 g) (SERVA, Heidelberg, Germany), sucrose (11.6 g) (Merck, Darmstadt, Germany), fresh yeast (28.33 g), and cornmeal (33.33 g) (both from local grocery store) in nanopure water (1 L), and heated to 60°C for 5 h under stirring. Methyl 4-hydroxybenzoate (NIPAGIN, Sigma-Aldrich Chemie GmbH, Steinheim, Germany) (0.83 g) dissolved in ethanol (8.33 mL) was subsequently added to sterilize the diet before its distribution into the vials.

### Sweet taste assay with D. melanogaster

The adult flies laid the eggs for 24 h and then they were transferred into new vials. The vials were incubated as described above. After 5-6 days, the third instar larvae became visible. In order to take the larvae from the vials, 50 mL of 10-20% sucrose solution was added and allowed to stand for 20 min. Larvae floated to the top. Next, the population was washed, three times, with deionized H₂O before the trials. Finally, the animals were collected in clean and empty vials. Subsequently, the assay chamber, a standard 35 mm x 15 mm plastic petri dish (ISOLAB GmbH, Wertheim, Germany), was prepared in the following manner: 20 mL of a 1% agarose (NIPPON Genetics Europe, Düren, Germany) solution was poured into the petri dish. Once the agarose was solid, the assay chambers were ready. All tastings were prepared on the same day. The assays were performed at the DanioVision observation chamber (Noldus, Wageningen, Netherlands). To start the observation, 100 µL of each tastant was added to the assay chamber with the testing larvae. As tastants were used: commercial Thaumatin, from *Thaumatococcus daniellii* Benth (Tokyo Chemical Industry, TCI), sucrose, MNEI, and Mabinllin II expressed based on the above protocol. Thaumatin was diluted in water. MNEI and Mabinlin were in 5 mM sodium phosphate pH 7.4. Larvae preference between water and 5 mM sodium phosphate buffer (pH 7.4) was evaluated, and the results showed that the larvae did not show any preference among them (data not shown).

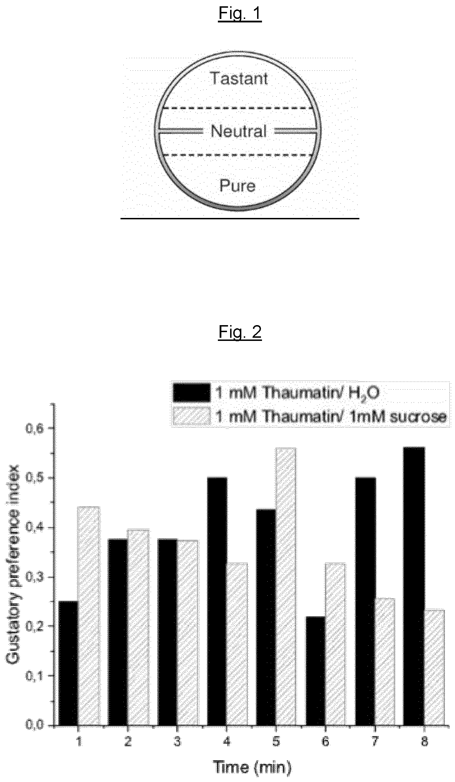

Then, the petri dish was placed in the DanioVision. The recording lasted 8 min. The plate was divided into three zones, the "tastant" zone, the "pure" zone, and a 1 cm wide "neutral" middle stripe **(****Fig. 1****).**

### Fig. 1. Schematic representation of the zones in the petri dish used in the behavioral assay (i. e. method for identification and/or measuring of sweetness).

For the calculation of the gustatory preference index (PREF), the number of larvae on the pure side (#pure) was subtracted from the number of larvae on the tastant side (#tastant) and divided this difference in the total number of larvae (#total), formula [1]: $PREF = \left(#tastant-#pure\right) / # total$

The PREF values were between 1 and -1, positive values indicating preference for and negative values indicating aversion of the tastant. The easy handling of larvae allowed to work with 10-15 animals simultaneously in one plate, maintaining the rest of the experimental conditions identical. Several plates were analyzed and the preference of the larvae was analyzed as one population.

### Results

### Establishment of the sweet taste assay

To establish a sweet taste assay and verify the sweetness thaumatin and third-instar larvae of *D. melanogaster* were used. The first attempt was performed with thaumatin (1 mM) against water (33 larvae) and against sucrose (1 mM, 43 larvae) **(****Fig. 2****).** This concentration from the sweet protein caused a strong attraction to larvae, scoring a positive gustatory preference index at all time points. The larvae not only perceived the sweet taste of thaumatin against water, but they exhibited a preference even compared to equal concentration of sucrose.

**Fig 2****. Gustatory choice of third instar larvae using thaumatin as tastant,** either against water (black) or against sucrose (striped). Gustatory preference indexes were calculated based on the distribution of larvae in the different zones, 1 - 8 min after assay onset.

### Expression and purification of proteins of interest

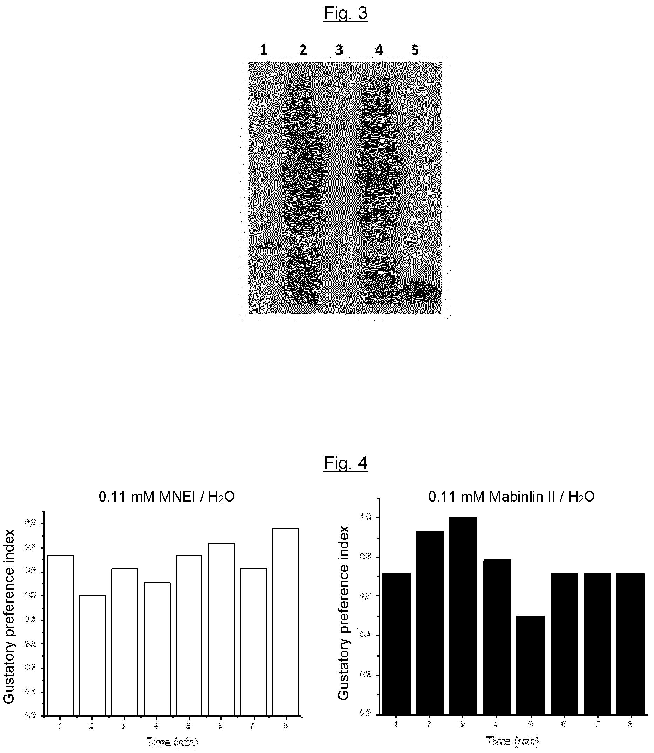

Apart from the commercial thaumatin, we decided to recombinantly express two sweet proteins in *E*. *coli,* in order to validate if the assay would be proven useful with proteins produced in small scale from such expression systems, in order to have a first quick assay before investing time and effort to go to larger production, in order to be able to support taste panel with sufficient amount of protein. As shown in **Fig. 3** MNEI and mabinlin-II were successfully expressed in *E. coli* BL21(DE3) and SHuffle T7, respectively. The yield of MNEI was 0.72 mg/L of cultivation and for mabinlin-II 2.1 mg/L.

**Fig. 3****. SDS-PAGE (15%) analysis of the proteins of interest.** 1) purified mabinlin-II (17 kDa); 2) flow-thought fraction from the purification of mabinlin-II; 3) purified MNEI (12 kDa); 4) flow-through fraction from the purification of MNEI, 8) Lysozyme (14.4 kDa), serving as standard.

### Validation of the sweet taste assay

After MNEI and mabinlin-II were heterologous produced, they were used in the sweet taste assay with 18 larvae each. MNEI and mabinlin-II were tested against water. Consistent with the earlier results from thaumatin, the animals showed a definite trend in favor of sweet proteins MNEI and Mabinlin-II **(****Fig. 4****),** even though these proteins were used in significantly lower concentrations compared to the thaumatin experiment (0.11 mM compared to 1 mM).

**Fig. 4****. Gustatory choice of third instar larvae using MNEI (left diagram) or mabinlin-II (right diagram) as tastant, against water.** Gustatory preference indexes were calculated based on the distribution of larvae in the different zones, 1 - 8 min after assay onset.

### Discussion

*Drosophila melanogaster,* despite having significant differences in the sweet taste receptor, perceives the taste very similar to humans. Like mammals, *Drosophila* species gustatory receptor (GRs) genes detect basic tastes comprising sweet, bitter, and salty. *Drosophila* taste receptors have seven transmembrane domains instead of three GPCRs, and they are distantly associated with *Drosophila* olfactory receptors (ORs). Until now, 68 GRs genes have been found in *Drosophila,* with Gr5a, Gr43a, Gr64a, and Gr64f being responsible for the sweet sensation. The Gr64a detects sucrose and maltose, and the Gr5a the trehalose and melezitose. The role of Gr64f is cooperative for the responses for all sugars tested with Gr5a and Gr64a. Gr43a is the only receptor known to detect fructose. In larvae, only Gr43a seems to have a critical role in taste recognition. This grants the advantage of using these insects for the validation of the sweetness of the heterologous expressed proteins. In addition, the insects are small, producing many progenies, have rapid growth, and perceive the taste very similar to humans are crucial advantages of housing *Drosophila* sp. This granting the advantage of using these insects to validate of the sweetness new candidates, which could be heterologous expressed proteins. In addition, to their human-like taste perception, the small size of these insects, their ability to produce many offspring, their rapid growth, and their close taste resemblance to humans are crucial advantages of working with *Drosophila.* In this work, a sweetness test using *D. melanogaster* larvae was established, allowing the calculation of the gustatory preference index.

The results from the aforementioned assays prove that the larvae can differentiate the sweet proteins from the solvent ("tastant" from "pure" zone), but also that they even exhibit preference for the thaumatin compared to sucrose, something that shows that the assay does not only give a qualitative analysis ("potentially sweet or not"), but it can also be used at least semi-quantitative, for estimation of sweetness factor, by applying different concentrations of the two sweet molecules and finding the ratio where the larvae will not show any preference. Based on the equation, the relative sweetness for the putative proteins is calculated, giving a quick assessment.

To validate we expressed recombinantly in *E*. *coli* two sweet proteins; MNEI and mabinlin-II. However, despite investigating different lysis buffers, including EDTA, higher salt concentrations, or surfactant, we could not identify a better lysis buffer; however, it seems that the proper dilution of the sample plays a more crucial role. Nevertheless, different lysis buffers or lysis conditions may be used without leaving the scope of the invention. With the assay, revealed herein, we clearly show, that the *D. melanogaster* larvae are able to detect low amounts of sweet proteins, for example such as MNEI and Mabinlin-II, both at 0.11 mM concentration. Even with some impurities from *E*. *coli* lysate, the flies were attracted towards the tastant zone, demonstrating the general applicability of our assay, both for low concentration samples, but also to samples with some impurities. Overall, it can be stated that the *D. melanogaster* sweet taste assay can be used for new potentially sweet proteins in low amounts, which granting the advantage that the proteins may not be isolated or produced in high yields. This would facilitate the interrogation of small to medium size libraries or potentially sweet proteins.

### Conclusion

The utilization of *D. melanogaster* larvae in this invention, owing to their human-like taste perception, small size, prolific reproduction, and rapid growth, presents a valuable model for validating the sweetness of proteins. This innovative assay not only reduces the need for large-scale taste experiments involving trained panelists but also allows screening of multiple samples in parallel with medium throughput for the identification of potentially novel sweet proteins. Moreover, it removes the barrier of the FDA or EFSA guidance the evaluation of human tasting panels, which states that experiments should only be performed after a safety evaluation, which can be cumbersome, if a large number of new proteins should be tested. The invention provides a very versatile new means for identification of novel sweet proteins and showcases the potential of the *D. melanogaster* larval assay in the quest for new sweet variants.

The core of the invention may be summarized as follows.

The invention comprises a method for identification and/or measuring of sweetness of compounds comprising the following steps:
- Providing larvae of a species of the family of Drosophilidae and/or of the family of Ephydroidea. It does not matter which larval stages (also known as instars) are used. Any larval stage can be used within the scope of the invention.
- Providing an observation chamber having two separate regions. The first region is to receive a reference compound and the second region is to receive the sample which is to be tested for sweetness or be measured for its contend of a sweet compound.
- Populating the observation chamber with the larvae provided according to first step and placing the reference compound and the sample each one into the region ascertained to it within the observation chamber. The succession of (1) populating with larvae, (2) placing reference compound and (3) placing sample is arbitrary. The larvae may even be raised to a certain stage of development while being already placed within the observation chamber before the sample is introduced into the observation chamber; by doing so, the observation chamber is also functioning as a growth chamber before observation takes place.

- Observing the behavior of the larvae as to within which of the two separate regions they prefer to stay. This observation may take different amounts of time, from a few seconds until some hours.
- Calculating a quantitative value from the observations of previous step (observing the behavior of the larvae). The result of the calculation will provide either a distinct decision if the sample exerts sweetness or will provide a numerical value of concentration of a sweet compound within the sample. The calculation is - for example - done by use of equation formula [1].

The method (as described just above) is preferredly executed by use of larvae of Drosophila melanogaster, strain Oregon R, but larvae of other species of Drosophilidae and/or Ephydroidea may also be used without leaving the scope of the invention.

The method as is described above is applicable to all compounds having sweetness, especially sugars and sweet proteins, but also for other compounds (e.g. synthetic sweeteners) or mixtures of compounds (e.g. stevia). Any sample containing at least one compound exerting sweetness can be measured.

Thus, the invention further comprises the usage of the method for evaluating differences in the sweetness of compounds within numerous types of mixtures, e.g.:
1.) Reaction mixtures;
2.) Food samples;
3.) Food extracts;
4.) Additives for food and feed;
5.) Extracts from organisms, for example Escherichia coli or Komagataella phaffii;
6.) Cell culture supernatants, for example Human embryonic kidney cells 293.

### Examples of sequences of recombinantly expressed proteins of interest

Mabinlin-II encoding gene with C-terminal 6xHis-tag
   ATGAGCATTC AGACCACCGT GATCGAAGTT GACGAGGAAG AGGATAACCA ACTGTGGCGT TGCCAGCGTC AATTCCTGCA GCACCAACGT CTGCGTGCGT GCCAGCGTTT CATTCACCGT CGTGCGCAGT TTGGTGGCCA ACCGGACGAA CTGGAGGATG AAGTGGAGGA CGATAACGAC GATGAGAACC AACCGCGTCG TCCGGCGCTG CGTCAATGCT GCAACCAGCT GCGTCAAGTT GACCGTCCGT GCGTGTGCCC GGTTCTGCGT CAGGCGGCGC AGCAAGTTCT GCAGCGTCAA ATCATTCAAG GTCCGCAGCA ACTGCGTCGT CTGTTCGATG CGGCGCGTAA CCTGCCGAAC ATCTGCAACA TTCCGAACAT CGGCACCTGC CCGTTTCGTA CCTGGCCGCT CGAGCACCAC CACCACCACC AC
Mabinlin-II with C-terminal 6xHis-tag
   MSIQTTVIEV DEEEDNQLWR CQRQFLQHQR LRACQRFIHR RAQFGGQPDE LEDEVEDDND DENQPRRPAL RQCCNQLRQV DRPCVCPVLR QAAQQVLQRQ IIQGPQQLRR LFDAARNLPN ICNIPNIGTC PFRTWPLEHH HHHH
MNEI encoding gene with C-terminal 6xHis-tag
   ATGGGTGAGT GGGAAATCAT TGACATCGGT CCGTTCACCC AGAACCTGGG CAAGTTTGCG GTGGATGAGG AAAACAAAAT TGGTCAATAC GGCCGTCTGA CCTTCAACAA GGTTATCCGT CCGTGCATGA AGAAAACCAT TTATGAGAAC GAAGGTTTTC GTGAGATCAA GGGCTACGAA TATCAGCTGT ACGTGTATGC GAGCGACAAA CTGTTCCGTG CGGACATTAG CGAGGATTAC AAGACCCGTG GTCGTAAACT GCTGCGTTTT AACGGCCCGG TGCCGCCGCC GCTCGAGCAC CACCACCACC ACCAC
MNEI with C-terminal 6xHis-tag
   MGEWEIIDIG PFTQNLGKFA VDEENKIGQY GRLTFNKVIR PCMKKTIYEN EGFREIKGYE YQLYVYASDK LFRADISEDY KTRGRKLLRF NGPVPPPLEH HHHHH

### List of lysis buffers tested and Figure of the SDS-PAGE analysis

Lysis buffer 1: Potassium phosphate buffer (50 mM, pH 8.0), 100 mM NaCl, 20% Glycerol Lysis buffer 2: Lysis buffer 1 + 100 mM NaCl
Lysis buffer 3: Lysis buffer 1 + 0.1% Triton X-100
Lysis buffer 4: Lysis buffer 1 + 1 mM EDTA
Lysis buffer 5: Lysis buffer 4 + 0.1% Triton X-100

SDS-PAGE (15%) of Mabinlin-II (17.2 kDa), expressed in E.coli SHuffle T7 cells, using different Lysis Buffer to resuspend before the sonication treatment:
1) marker,
2) soluble with lysis buffer 1,
3) pellet with lysis buffer 1,
4) soluble with lysis buffer 2,
5) pellet with lysis buffer 2,
6) soluble with lysis buffer 3,
7) pellet with lysis buffer 3,
8) soluble with lysis buffer 4,
9) pellet with lysis buffer 4,
10) soluble with lysis buffer 5,
11) pellet with lysis buffer 5

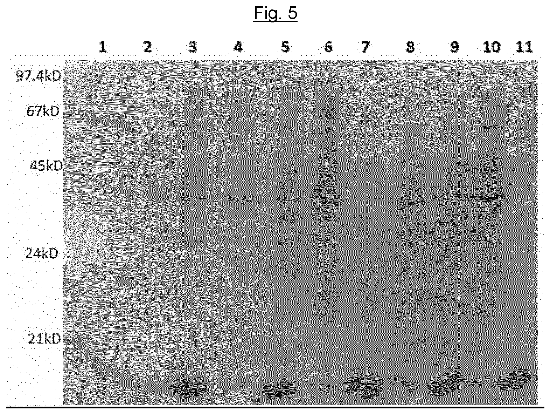

Fig. 5. SDS-PAGE analysis

## Claims

1. A method for identification and/or measuring of sweetness of compounds comprising the steps
a) providing larvae of a species of the family of Drosophilidae and/or of the family of Ephydroidea;
b) providing an observation chamber having two separate regions, whereat the first region is to receive a reference compound and the second region is to receive the sample which is to be tested for sweetness or be measured for its contend of a sweet compound;
c) populating the observation chamber with the larvae provided according to step a) and placing the reference compound and the sample each one into the region ascertained to it within the observation chamber, whereat the succession of populating with larvae, placing reference compound and placing sample is arbitrary; .
d) observing the behavior of the larvae as to within which of the two separate regions they prefer to stay;
e) calculating a quantitative value from the observations of step d), thereby providing either a distinct decision if the sample exerts sweetness or providing a numerical value of concentration of a sweet compound within the sample.

2. The method according to claim 1, **characterized in that** the larvae provided in step a) are larvae of Drosophila melanogaster, strain Oregon R.

3. The method according to claim 1 or claim 2, **characterized in that** the sample comprises at least one sugar or at least one sweet protein or a combination of at least one sugar with at least one sweet protein.

4. Usage of the method according to claim 1 or claim 2 or claim 3 for evaluating differences in the sweetness of compounds within:
i) Reaction mixtures or
ii) Food samples or
iii) Food extracts or
iv) Additives for food and feed or
v) Extracts from organisms, for example Escherichia coli or Komagataella phaffii
vi) Cell culture supernatants, for example Human embryonic kidney cells 293.
